# EUROPEAN PATENT APPLICATION

(11) **EP 4 834 134 A2**
(43) Date of publication of application: **04.11.2026**
(21) Application number: 26198139.3
(22) Date of filing: 31.10.2019
(51) Int. Cl.: A24F 40/10

(54) **AEROSOLISED FORMULATION**

(30) Priority: 01.11.2018 GB 201817868
(62) Document number(s) of the earlier application(s) in accordance with Art. 76 EPC: 19798366.1 / 3 873 246
(71) Applicant: Nicoventures Trading Limited, London WC2R 3LA (GB)
(72) Inventor: CABOT, Ross, London, WC2R 3LA (GB)
(74) Representative: D Young & Co LLP

(57) **Abstract**

There is provided an aerosolised formulation comprising
(i) water in an amount of at least 50 wt.% based on the aerosolised formulation; and
(ii) at least one active agent;
wherein the aerosol has the following particle size distribution: D50 of from 2 to 6µm.

## Description

### FIELD OF THE INVENTION

The present disclosure relates to an aerosolised formulation, a method of forming the same, a device for forming the same and processes and uses of the same.

### BACKGROUND TO THE INVENTION

Electronic aerosol provision systems such as e-cigarettes generally contain a reservoir of liquid which is to be vaporised, typically containing a flavour or an active agent such as nicotine. When a user inhales on the device, a heater is activated to vaporise a small amount of liquid, which is therefore inhaled by the user.

The use of e-cigarettes in the UK has grown rapidly, and it has been estimated that there are now over a million people using them in the UK.

One challenge faced in providing such systems is to provide from the aerosol provision device a aerosol to be inhaled which provides consumers with an acceptable experience. Some consumers may prefer an e-cigarette that generates an aerosol that closely 'mimics' smoke inhaled from a tobacco product such as a cigarette. Aerosols from e-cigarettes and smoke from tobacco products such as cigarettes provides to the user a complex chain of flavour in the mouth, and if nicotine is present, nicotine absorption in the mouth and throat, followed by nicotine absorption in the lungs. These various aspects are described by users in terms of flavour, intensity/quality, impact, irritation/smoothness and reward. Flavour contributes to a number of these factors, and is strongly associated with flavour in the mouth and the provision of desirable taste and smell, whether mimicking the taste and smell of a tobacco product or providing alternative flavours. Reliably providing a particular taste and smell is made more difficult by the volatile and thermally sensitive nature of some flavours. Heating of flavour components in e-cigarettes may result in some flavours being degraded. This has a number of disadvantages. Flavours present in the liquid may be lost resulting in a diminished flavour experience for the user or the need to include in the liquid excess flavour at additional cost. Furthermore if e-liquid contains multiple flavours and only some of these multiple flavours are degraded, this can adversely affect the balance of the taste and smell. Furthermore, degraded flavours may have an undesirable or "off taste". Each of these factors, and their balance, can strongly contribute to consumer acceptability of an e-cigarette. Providing means to optimise the overall vaping experience is therefore desirable to e-cigarette manufacturers.

A further challenge facing such systems is the continued demand for harm reduction. Harm from cigarette and e-cigarette devices primarily comes from toxicants. Therefore, there is a desire to reduce the potential for the formation of toxicants.

### SUMMARY OF THE INVENTION

In one aspect there is provided an aerosolised formulation comprising
(i) water in an amount of at least 50 wt.% based on the aerosolised formulation; and
(ii) at least one active agent;
wherein the aerosol has the following particle size distribution: D50 of from 2 to 6µm.

In one aspect there is provided a process for forming an aerosol, the process comprising aerosolising an aerosolisable formulation comprising
(i) water in an amount of at least 50 wt.% based on the aerosolisable formulation; and
(ii) at least one active agent;
to provide an aerosol having the following particle size distribution: D50 of from 3 to 6µm.

In one aspect there is provided an electronic aerosol provision system comprising:
(a) an aerosoliser for aerosolising formulation for inhalation by a user of the electronic aerosol provision system;
(b) a power supply comprising a cell or battery for supplying power to the aerosoliser
(c) aerosolisable formulation comprising
   (i) water in an amount of at least 50 wt.% based on the aerosolisable formulation; and
   (ii) at least one active agent;
wherein the aerosoliser provides an aerosol having the following particle size distribution: D50 of from 2 to 6µm.

In one aspect there is provided a process for improving delivery of an aerosolised formulation to the lungs, the process comprising the steps of
(a) providing an aerosolisable material comprising (i) water in an amount of at least 50 wt.% based on the aerosolisable material and (ii) at least one active agent;
(b) aerosolising the material aerosolisable material to provide an aerosol having the following particle size distribution: D50 of from 2 to 6µm.

### DETAILED DESCRIPTION

As discussed herein in one aspect there is provided an aerosolised formulation comprising
(i) water in an amount of at least 50 wt.% based on the aerosolised formulation; and
(ii) at least one active agent;
wherein the aerosol has the following particle size distribution: D50 of from 2 to 6µm.

We have found that an advantageous system may be provided in which an aerosolised formulation containing a high content of water and an active agent is formed from an aerosolisable formulation. In particular, we have identified that a water-based system may be provided in which aerosolised formulation is formed from an aerosolisable formulation at a low temperature. This is in contrast to 'traditional' e-cigarettes which use a heater, typically applied to liquids based on glycerol and propylene glycol to form an aerosolised formulation. The provision of such a system and the avoidance of heating may address problems of the prior art relating to the formation of toxicants. More specifically, we have identified a water-based system which, by careful selection of the particle size distribution of the aerosol, delivers water droplets containing the active agent into desirable areas of the lungs, such as the deep lung. In particular, the aerosol has a D50 particle size distribution of from 2 to 6µm. We have identified that in water-based aerosols, the droplets have a tendency to evaporate and decrease in size when inhaled by the user. Therefore the specific selection of particle size described herein addresses the reduction of size through evaporation and still delivers the active agent into the desired area of the lungs. These problems and the choice made in the present invention is in contrast to the prior liquids based on glycerol and propylene glycol in which the liquid droplets tend to adhere to moisture within the lungs and thereby increase in size when inhaled.

For ease of reference, these and further aspects of the present invention are now discussed under appropriate section headings. However, the teachings under each section are not necessarily limited to each particular section.

### Water

As discussed herein the aerosolised formulation comprises water in an amount of at least 50 wt.% based on the aerosolised formulation. In one aspect water is present in an amount of at least 55 wt.% based on the aerosolised formulation. In one aspect water is present in an amount of at least 60 wt.% based on the aerosolised formulation. In one aspect water is present in an amount of at least 65 wt.% based on the aerosolised formulation. In one aspect water is present in an amount of at least 70 wt.% based on the aerosolised formulation. In one aspect water is present in an amount of at least 75 wt.% based on the aerosolised formulation. In one aspect water is present in an amount of at least 80 wt.% based on the aerosolised formulation. In one aspect water is present in an amount of at least 85 wt.% based on the aerosolised formulation. In one aspect water is present in an amount of at least 90 wt.% based on the aerosolised formulation. In one aspect water is present in an amount of at least 95 wt.% based on the aerosolised formulation. In one aspect water is present in an amount of at least 99 wt.% based on the aerosolised formulation.

In one aspect water is present in an amount of from 50 to 99 wt.% based on the aerosolised formulation. In one aspect water is present in an amount of from 55 to 99 wt.% based on the aerosolised formulation. In one aspect water is present in an amount of from 60 to 99 wt.% based on the aerosolised formulation. In one aspect water is present in an amount of from 65 to 99 wt.% based on the aerosolised formulation. In one aspect water is present in an amount of from 70 to 99 wt.% based on the aerosolised formulation. In one aspect water is present in an amount of from 75 to 99 wt.% based on the aerosolised formulation. In one aspect water is present in an amount of from 80 to 99 wt.% based on the aerosolised formulation. In one aspect water is present in an amount of from 85 to 99 wt.% based on the aerosolised formulation. In one aspect water is present in an amount of from 90 to 99 wt.% based on the aerosolised formulation. In one aspect water is present in an amount of from 95 to 99 wt.% based on the aerosolised formulation.

As discussed herein the aerosolisable formulation comprises water in an amount of at least 50 wt.% based on the aerosolisable formulation. In one aspect water is present in an amount of at least 55 wt.% based on the aerosolisable formulation. In one aspect water is present in an amount of at least 60 wt.% based on the aerosolisable formulation. In one aspect water is present in an amount of at least 65 wt.% based on the aerosolisable formulation. In one aspect water is present in an amount of at least 70 wt.% based on the aerosolisable formulation. In one aspect water is present in an amount of at least 75 wt.% based on the aerosolisable formulation. In one aspect water is present in an amount of at least 80 wt.% based on the aerosolisable formulation. In one aspect water is present in an amount of at least 85 wt.% based on the aerosolisable formulation. In one aspect water is present in an amount of at least 90 wt.% based on the aerosolisable formulation. In one aspect water is present in an amount of at least 95 wt.% based on the aerosolisable formulation. In one aspect water is present in an amount of at least 99 wt.% based on the aerosolisable formulation.

In one aspect water is present in an amount of from 50 to 99 wt.% based on the aerosolisable formulation. In one aspect water is present in an amount of from 55 to 99 wt.% based on the aerosolisable formulation. In one aspect water is present in an amount of from 60 to 99 wt.% based on the aerosolisable formulation. In one aspect water is present in an amount of from 65 to 99 wt.% based on the aerosolisable formulation. In one aspect water is present in an amount of from 70 to 99 wt.% based on the aerosolisable formulation. In one aspect water is present in an amount of from 75 to 99 wt.% based on the aerosolisable formulation. In one aspect water is present in an amount of from 80 to 99 wt.% based on the aerosolisable formulation. In one aspect water is present in an amount of from 85 to 99 wt.% based on the aerosolisable formulation. In one aspect water is present in an amount of from 90 to 99 wt.% based on the aerosolisable formulation. In one aspect water is present in an amount of from 95 to 99 wt.% based on the aerosolisable formulation.

As discussed herein the use of water allows for the replacement of some or all of the glycerol, propylene glycol, 1,3-propane diol and mixtures thereof typically used in e-cigarettes. In one aspect the aerosolisable formulation contains glycerol, propylene glycol, 1,3-propane diol and mixtures thereof in a combined amount of no greater than 10 wt.% based on the aerosolisable formulation or based on the aerosolised formulation. In one aspect the aerosolisable formulation contains glycerol, propylene glycol, 1,3-propane diol and mixtures thereof in a combined amount of no greater than 8 wt.% based on the aerosolisable formulation or based on the aerosolised formulation. In one aspect the aerosolisable formulation contains glycerol, propylene glycol, 1,3-propane diol and mixtures thereof in a combined amount of no greater than 5 wt.% based on the aerosolisable formulation or based on the aerosolised formulation. In one aspect the aerosolisable formulation contains glycerol, propylene glycol, 1,3-propane diol and mixtures thereof in a combined amount of no greater than 2 wt.% based on the aerosolisable formulation or based on the aerosolised formulation. In one aspect the aerosolisable formulation contains glycerol, propylene glycol, 1,3-propane diol and mixtures thereof in a combined amount of no greater than 1 wt.% based on the aerosolisable formulation or based on the aerosolised formulation. In one aspect the aerosolisable formulation contains glycerol, propylene glycol, 1,3-propane diol and mixtures thereof in a combined amount of no greater than 0.5 wt.% based on the aerosolisable formulation or based on the aerosolised formulation. In one aspect the aerosolisable formulation contains glycerol, propylene glycol, 1,3-propane diol and mixtures thereof in a combined amount of no greater than 0.2 wt.% based on the aerosolisable formulation or based on the aerosolised formulation. In one aspect the aerosolisable formulation contains glycerol, propylene glycol, 1,3-propane diol and mixtures thereof in a combined amount of no greater than 0.1 wt.% based on the aerosolisable formulation or based on the aerosolised formulation. In one aspect the aerosolisable formulation contains glycerol, propylene glycol, 1,3-propane diol and mixtures thereof in a combined amount of no greater than 0.01 wt.% based on the aerosolisable formulation or based on the aerosolised formulation. In one aspect the aerosolisable formulation contains no glycerol, propylene glycol, 1,3-propane diol and mixtures thereof.

In one aspect the aerosolisable formulation contains glycerol, propylene glycol, and mixtures thereof in a combined amount of no greater than 10 wt.% based on the aerosolisable formulation or based on the aerosolised formulation. In one aspect the aerosolisable formulation contains glycerol, propylene glycol, and mixtures thereof in a combined amount of no greater than 8 wt.% based on the aerosolisable formulation or based on the aerosolised formulation. In one aspect the aerosolisable formulation contains glycerol, propylene glycol, and mixtures thereof in a combined amount of no greater than 5 wt.% based on the aerosolisable formulation or based on the aerosolised formulation. In one aspect the aerosolisable formulation contains glycerol, propylene glycol, and mixtures thereof in a combined amount of no greater than 2 wt.% based on the aerosolisable formulation or based on the aerosolised formulation. In one aspect the aerosolisable formulation contains glycerol, propylene glycol, and mixtures thereof in a combined amount of no greater than 1 wt.% based on the aerosolisable formulation or based on the aerosolised formulation. In one aspect the aerosolisable formulation contains glycerol, propylene glycol, and mixtures thereof in a combined amount of no greater than 0.5 wt.% based on the aerosolisable formulation or based on the aerosolised formulation. In one aspect the aerosolisable formulation contains glycerol, propylene glycol, and mixtures thereof in a combined amount of no greater than 0.2 wt.% based on the aerosolisable formulation or based on the aerosolised formulation. In one aspect the aerosolisable formulation contains glycerol, propylene glycol, and mixtures thereof in a combined amount of no greater than 0.1 wt.% based on the aerosolisable formulation or based on the aerosolised formulation. In one aspect the aerosolisable formulation contains glycerol, propylene glycol, and mixtures thereof in a combined amount of no greater than 0.01 wt.% based on the aerosolisable formulation or based on the aerosolised formulation. In one aspect the aerosolisable formulation contains no glycerol, propylene glycol, and mixtures thereof.

In one aspect the aerosolisable formulation contains glycerol in an amount of no greater than 10 wt.% based on the aerosolisable formulation or based on the aerosolised formulation. In one aspect the aerosolisable formulation contains glycerol in an amount of no greater than 8 wt.% based on the aerosolisable formulation or based on the aerosolised formulation. In one aspect the aerosolisable formulation contains glycerol in an amount of no greater than 5 wt.% based on the aerosolisable formulation or based on the aerosolised formulation. In one aspect the aerosolisable formulation contains glycerol in an amount of no greater than 2 wt.% based on the aerosolisable formulation or based on the aerosolised formulation. In one aspect the aerosolisable formulation contains glycerol in an amount of no greater than 1 wt.% based on the aerosolisable formulation or based on the aerosolised formulation. In one aspect the aerosolisable formulation contains glycerol in an amount of no greater than 0.5 wt.% based on the aerosolisable formulation or based on the aerosolised formulation. In one aspect the aerosolisable formulation contains glycerol in an amount of no greater than 0.2 wt.% based on the aerosolisable formulation or based on the aerosolised formulation. In one aspect the aerosolisable formulation contains glycerol in an amount of no greater than 0.1 wt.% based on the aerosolisable formulation or based on the aerosolised formulation. In one aspect the aerosolisable formulation contains glycerol in an amount of no greater than 0.01 wt.% based on the aerosolisable formulation or based on the aerosolised formulation. In one aspect the aerosolisable formulation contains no glycerol.

In one aspect the aerosolisable formulation contains propylene glycol in an amount of no greater than 10 wt.% based on the aerosolisable formulation or based on the aerosolised formulation. In one aspect the aerosolisable formulation contains propylene glycol in an amount of no greater than 8 wt.% based on the aerosolisable formulation or based on the aerosolised formulation. In one aspect the aerosolisable formulation contains propylene glycol in an amount of no greater than 5 wt.% based on the aerosolisable formulation or based on the aerosolised formulation. In one aspect the aerosolisable formulation contains propylene glycol in an amount of no greater than 2 wt.% based on the aerosolisable formulation or based on the aerosolised formulation. In one aspect the aerosolisable formulation contains propylene glycol in an amount of no greater than 1 wt.% based on the aerosolisable formulation or based on the aerosolised formulation. In one aspect the aerosolisable formulation contains propylene glycol in an amount of no greater than 0.5 wt.% based on the aerosolisable formulation or based on the aerosolised formulation. In one aspect the aerosolisable formulation contains propylene glycol in an amount of no greater than 0.2 wt.% based on the aerosolisable formulation or based on the aerosolised formulation. In one aspect the aerosolisable formulation contains propylene glycol in an amount of no greater than 0.1 wt.% based on the aerosolisable formulation or based on the aerosolised formulation. In one aspect the aerosolisable formulation contains propylene glycol in an amount of no greater than 0.01 wt.% based on the aerosolisable formulation or based on the aerosolised formulation. In one aspect the aerosolisable formulation contains no propylene glycol.

### Active Agent

The aerosolisable formulation and the aerosolised formulation further comprise an active agent. By "active agent" it is meant an agent which has a biological effect, such as sensorial and/or physiological effect, on a subject when the aerosol is inhaled. In one aspect by "active agent" it is meant an agent which has a physiological effect on a subject when the aerosol is inhaled. In some cases, the active may be a flavour. The active agent may for example be selected from nutraceuticals, nootropics, psychoactives. The active substance may be naturally occurring or synthetically obtained. The active agent may comprise for example nicotine, caffeine, taurine, theine, vitamins such as B6 or B12 or C, melatonin, cannabinoids, or constituents, derivatives, or combinations thereof. The active agent may comprise one or more constituents, derivatives or extracts of tobacco, cannabis or another botanical. In some embodiments, the active agent comprises caffeine, melatonin or vitamin B12. The one or more active agents may be selected from nicotine, botanicals, and mixtures thereof. The one or more active agents may be of synthetic or natural origin. The active could be an extract from a botanical, such as from a plant in the tobacco family. In one aspect the active agent is at least nicotine. In one aspect the active agent consists of nicotine and/or salts thereof.

As noted herein, the active agent may comprise one or more constituents, derivatives or extracts of cannabis, such as one or more cannabinoids or terpenes. Cannabinoids are a class of natural or synthetic chemical compounds which act on cannabinoid receptors (i.e., CB1 and CB2) in cells that repress neurotransmitter release in the brain. Cannabinoids may be naturally occurring (phytocannabinoids) from plants such as cannabis, from animals (endocannabinoids), or artificially manufactured (synthetic cannabinoids). Cannabis species express at least 85 different phytocannabinoids, and are divided into subclasses, including cannabigerols, cannabichromenes, cannabidiols, tetrahydrocannabinols, cannabinols and cannabinodiols, and other cannabinoids. Cannabinoids found in cannabis include, without limitation: cannabigerol (CBG), cannabichromene (CBC), cannabidiol (CBD), tetrahydrocannabinol (THC), cannabinol (CBN), cannabinodiol (CBDL), cannabicyclol (CBL), cannabivarin (CBV), tetrahydrocannabivarin (THCV), cannabidivarin (CBDV), cannabichromevarin (CBCV), cannabigerovarin (CBGV), cannabigerol monomethyl ether (CBGM), cannabinerolic acid, cannabidiolic acid (CBDA), Cannabinol propyl variant (CBNV), cannabitriol (CBO), tetrahydrocannabmolic acid (THCA), and tetrahydrocannabivarinic acid (THCV A).

In some embodiments, the active agent comprises one or more constituents, derivatives or extracts of cannabis.

As noted herein, the active agent may comprise or be derived from one or more botanicals or constituents, derivatives or extracts thereof. As used herein, the term "botanical" includes any material derived from plants including, but not limited to, extracts, leaves, bark, fibres, stems, roots, seeds, flowers, fruits, pollen, husk, shells or the like. Alternatively, the material may comprise an active compound naturally existing in a botanical, obtained synthetically. The material may be in the form of liquid, gas, solid, powder, dust, crushed particles, granules, pellets, shreds, strips, sheets, or the like. Example botanicals are tobacco, eucalyptus, star anise, hemp, cocoa, cannabis, fennel, lemongrass, peppermint, spearmint, rooibos, chamomile, flax, ginger, ginkgo biloba, hazel, hibiscus, laurel, licorice (liquorice), matcha, mate, orange skin, papaya, rose, sage, tea such as green tea or black tea, thyme, clove, cinnamon, coffee, aniseed (anise), basil, bay leaves, cardamom, coriander, cumin, nutmeg, oregano, paprika, rosemary, saffron, lavender, lemon peel, mint, juniper, elderflower, vanilla, wintergreen, beefsteak plant, curcuma, turmeric, sandalwood, cilantro, bergamot, orange blossom, myrtle, cassis, valerian, pimento, mace, damien, marjoram, olive, lemon balm, lemon basil, chive, carvi, verbena, tarragon, geranium, mulberry, ginseng, theanine, theacrine, maca, ashwagandha, damiana, guarana, chlorophyll, baobab or any combination thereof. The mint may be chosen from the following mint varieties: Mentha Arventis, Mentha c.v.,Mentha niliaca, Mentha piperita, Mentha piperita citrata c.v.,Mentha piperita c.v, Mentha spicata crispa, Mentha cardifolia, Memtha longifolia, Mentha suaveolens variegata, Mentha pulegium, Mentha spicata c.v. and Mentha suaveolens.

In some embodiments, the active agent comprises or derived from one or more botanicals or constituents, derivatives or extracts thereof and the botanical is tobacco.

In some embodiments, the active agent comprises or derived from one or more botanicals or constituents, derivatives or extracts thereof and the botanical is selected from eucalyptus, star anise, cocoa and hemp.

In some embodiments, the active agent comprises or derived from one or more botanicals or constituents, derivatives or extracts thereof and the botanical is selected from rooibos and fennel.

In one aspect the one or more active agents are present in a combined amount (such as nicotine is present in an amount) of no greater than 6 wt% based on the aerosolisable formulation or based on the aerosolised formulation. In one aspect the one or more active agents are present in a combined amount (such as nicotine is present in an amount) of from 0.01 to 6 wt% based on the aerosolisable formulation or based on the aerosolised formulation. In one aspect the one or more active agents are present in a combined amount (such as nicotine is present in an amount) of from 0.02 to 6 wt% based on the aerosolisable formulation or based on the aerosolised formulation. In one aspect the one or more active agents are present in a combined amount (such as nicotine is present in an amount) of from 0.05 to 6 wt% based on the aerosolisable formulation or based on the aerosolised formulation. In one aspect the one or more active agents are present in a combined amount (such as nicotine is present in an amount) of from 0.08 to 6 wt% based on the aerosolisable formulation or based on the aerosolised formulation. In one aspect the one or more active agents are present in a combined amount (such as nicotine is present in an amount) of from 0.01 to 5 wt% based on the aerosolisable formulation or based on the aerosolised formulation. In one aspect the one or more active agents are present in a combined amount (such as nicotine is present in an amount) of from 0.02 to 5 wt% based on the aerosolisable formulation or based on the aerosolised formulation. In one aspect the one or more active agents are present in a combined amount (such as nicotine is present in an amount) of from 0.05 to 5 wt% based on the aerosolisable formulation or based on the aerosolised formulation. In one aspect the one or more active agents are present in a combined amount (such as nicotine is present in an amount) of from 0.08 to 5 wt% based on the aerosolisable formulation or based on the aerosolised formulation. In one aspect the one or more active agents are present in a combined amount (such as nicotine is present in an amount) of no greater than 4 wt% based on the aerosolisable formulation or based on the aerosolised formulation. In one aspect the one or more active agents are present in a combined amount (such as nicotine is present in an amount) of from 0.01 to 4 wt% based on the aerosolisable formulation or based on the aerosolised formulation. In one aspect the one or more active agents are present in a combined amount (such as nicotine is present in an amount) of from 0.02 to 4 wt% based on the aerosolisable formulation or based on the aerosolised formulation. In one aspect the one or more active agents are present in a combined amount (such as nicotine is present in an amount) of from 0.05 to 4 wt% based on the aerosolisable formulation or based on the aerosolised formulation. In one aspect the one or more active agents are present in a combined amount (such as nicotine is present in an amount) of from 0.08 to 4 wt% based on the aerosolisable formulation or based on the aerosolised formulation. In one aspect the one or more active agents are present in a combined amount (such as nicotine is present in an amount) of no greater than 3 wt% based on the aerosolisable formulation or based on the aerosolised formulation. In one aspect the one or more active agents are present in a combined amount (such as nicotine is present in an amount) of from 0.01 to 3 wt% based on the aerosolisable formulation or based on the aerosolised formulation. In one aspect the one or more active agents are present in a combined amount (such as nicotine is present in an amount) of from 0.02 to 3 wt% based on the aerosolisable formulation or based on the aerosolised formulation. In one aspect the one or more active agents are present in a combined amount (such as nicotine is present in an amount) of from 0.05 to 3 wt% based on the aerosolisable formulation or based on the aerosolised formulation. In one aspect the one or more active agents are present in a combined amount (such as nicotine is present in an amount) of from 0.08 to 3 wt% based on the aerosolisable formulation or based on the aerosolised formulation. In one aspect the one or more active agents are present in a combined amount (such as nicotine is present in an amount) of no greater than 2 wt% based on the aerosolisable formulation or based on the aerosolised formulation. In one aspect the one or more active agents are present in a combined amount (such as nicotine is present in an amount) of from 0.01 to 2 wt% based on the aerosolisable formulation or based on the aerosolised formulation. In one aspect the one or more active agents are present in a combined amount (such as nicotine is present in an amount) of from 0.02 to 2 wt% based on the aerosolisable formulation or based on the aerosolised formulation. In one aspect the one or more active agents are present in a combined amount (such as nicotine is present in an amount) of from 0.05 to 2 wt% based on the aerosolisable formulation or based on the aerosolised formulation. In one aspect the one or more active agents are present in a combined amount (such as nicotine is present in an amount) of from 0.08 to 2 wt% based on the aerosolisable formulation or based on the aerosolised formulation. In one aspect the one or more active agents are present in a combined amount (such as nicotine is present in an amount) of no greater than 1 wt% based on the aerosolisable formulation or based on the aerosolised formulation. In one aspect the one or more active agents are present in a combined amount (such as nicotine is present in an amount) of from 0.01 to 1 wt% based on the aerosolisable formulation or based on the aerosolised formulation. In one aspect the one or more active agents are present in a combined amount (such as nicotine is present in an amount) of from 0.02 to 1 wt% based on the aerosolisable formulation or based on the aerosolised formulation. In one aspect the one or more active agents are present in a combined amount (such as nicotine is present in an amount) of from 0.05 to 1 wt% based on the aerosolisable formulation or based on the aerosolised formulation. In one aspect the one or more active agents are present in a combined amount (such as nicotine is present in an amount) of from 0.08 to 1 wt% based on the aerosolisable formulation or based on the aerosolised formulation. In one aspect the one or more active agents are present in a combined amount (such as nicotine is present in an amount) of from 0.1 to 1 wt% based on the aerosolisable formulation or based on the aerosolised formulation. In one aspect the one or more active agents are present in a combined amount (such as nicotine is present in an amount) of no greater than 0.6 wt% based on the aerosolisable formulation or based on the aerosolised formulation. In one aspect the one or more active agents are present in a combined amount (such as nicotine is present in an amount) of from 0.01 to 0.6 wt% based on the aerosolisable formulation or based on the aerosolised formulation. In one aspect the one or more active agents are present in a combined amount (such as nicotine is present in an amount) of from 0.02 to 0.6 wt% based on the aerosolisable formulation or based on the aerosolised formulation. In one aspect the one or more active agents are present in a combined amount (such as nicotine is present in an amount) of from 0.05 to 0.6 wt% based on the aerosolisable formulation or based on the aerosolised formulation. In one aspect the one or more active agents are present in a combined amount (such as nicotine is present in an amount) of from 0.08 to 0.6 wt% based on the aerosolisable formulation or based on the aerosolised formulation. In one aspect the one or more active agents are present in a combined amount (such as nicotine is present in an amount) of from 0.1 to 0.6 wt% based on the aerosolisable formulation or based on the aerosolised formulation. In one aspect the one or more active agents are present in a combined amount (such as nicotine is present in an amount) of no greater than 0.5 wt% based on the aerosolisable formulation or based on the aerosolised formulation. In one aspect the one or more active agents are present in a combined amount (such as nicotine is present in an amount) of from 0.01 to 0.5 wt% based on the aerosolisable formulation or based on the aerosolised formulation. In one aspect the one or more active agents are present in a combined amount (such as nicotine is present in an amount) of from 0.02 to 0.5 wt% based on the aerosolisable formulation or based on the aerosolised formulation. In one aspect the one or more active agents are present in a combined amount (such as nicotine is present in an amount) of from 0.05 to 0.5 wt% based on the aerosolisable formulation or based on the aerosolised formulation. In one aspect the one or more active agents are present in a combined amount (such as nicotine is present in an amount) of from 0.08 to 0.5 wt% based on the aerosolisable formulation or based on the aerosolised formulation. In one aspect the one or more active agents are present in a combined amount (such as nicotine is present in an amount) of no greater than 0.2 wt% based on the aerosolisable formulation or based on the aerosolised formulation. In one aspect the one or more active agents are present in a combined amount (such as nicotine is present in an amount) of from 0.01 to 0.2 wt% based on the aerosolisable formulation or based on the aerosolised formulation. In one aspect the one or more active agents are present in a combined amount (such as nicotine is present in an amount) of from 0.02 to 0.2 wt% based on the aerosolisable formulation or based on the aerosolised formulation. In one aspect the one or more active agents are present in a combined amount (such as nicotine is present in an amount) of from 0.05 to 0.2 wt% based on the aerosolisable formulation or based on the aerosolised formulation. In one aspect the one or more active agents are present in a combined amount (such as nicotine is present in an amount) of from 0.08 to 0.2 wt% based on the aerosolisable formulation or based on the aerosolised formulation. In one aspect the one or more active agents are present in a combined amount (such as nicotine is present in an amount) of no greater than 0.1 wt% based on the aerosolisable formulation or based on the aerosolised formulation. In one aspect the one or more active agents are present in a combined amount (such as nicotine is present in an amount) of from 0.01 to 0.1 wt% based on the aerosolisable formulation or based on the aerosolised formulation. In one aspect the one or more active agents are present in a combined amount (such as nicotine is present in an amount) of from 0.02 to 0.1 wt% based on the aerosolisable formulation or based on the aerosolised formulation. In one aspect the one or more active agents are present in a combined amount (such as nicotine is present in an amount) of from 0.05 to 0.1 wt% based on the aerosolisable formulation or based on the aerosolised formulation. In one aspect the one or more active agents are present in a combined amount (such as nicotine is present in an amount) of from 0.08 to 0.1 wt% based on the aerosolisable formulation or based on the aerosolised formulation.

### Particle Size Distribution

The aerosol of the aerosolised formulation has the following particle size distribution: D50 of from 2 to 6µm. References in the present specification to particle size distribution, D50, D10 or D90 refer to values measured in accordance with British and European Pharmacopoeia, 2.9.31 Particle Size Analysis By Laser Light Diffraction (see BRITISH PHARMACOPOEIA COMMISSION. (2014), British Pharmacopoeia. London, England: Stationery Office and COUNCIL OF EUROPE. (2013). European Pharmacopoeia. Strasbourg, France: Council of Europe). The terms D50, Dv50 and Dx50 are interchangeable. The terms D10, Dv10 and Dx10 are interchangeable. The terms D90, Dv90 and Dx90 are interchangeable.

In one aspect the aerosol has a D50 of from 2.5 to 6µm. In one aspect the aerosol has a D50 of from 3 to 6µm. In one aspect the aerosol has a D50 of from 3.5 to 6µm. In one aspect the aerosol has a D50 of from 4 to 6µm. In one aspect the aerosol has a D50 of from 4.5 to 6µm. In one aspect the aerosol has a D50 of from 5 to 6µm. In one aspect the aerosol has a D50 of from 2.5 to 5.5µm. In one aspect the aerosol has a D50 of from 3 to 5.5µm. In one aspect the aerosol has a D50 of from 3.5 to 5.5µm. In one aspect the aerosol has a D50 of from 4 to 5.5µm. In one aspect the aerosol has a D50 of from 4.5 to 5.5µm. In one aspect the aerosol has a D50 of from 5 to 5.5µm. In one aspect the aerosol has a D50 of from 4 to 5.5µm. In one aspect the aerosol has a D50 of from 4 to 5µm. In one aspect the aerosol has a D50 of from 4 to 4.5µm. In one aspect the aerosol has a D50 of from 2.5 to 5µm. In one aspect the aerosol has a D50 of from 2.5 to 4.5µm.

In one aspect the aerosol has a D10 of at least 0.5µm. In one aspect the aerosol has a D10 of at least 1µm. In one aspect the aerosol has a D10 of at least 1.5µm. In one aspect the aerosol has a D10 of at least 2µm.

In one aspect the aerosol has a D90 of no greater than 15µm. In one aspect the aerosol has a D90 of no greater than 12µm. In one aspect the aerosol has a D90 of no greater than 10µm.

In one aspect D50 is measured after exclusion of particles having a particle size of less than 1µm. In one aspect D10 is measured after exclusion of particles having a particle size of less than 1µm. In one aspect D90 is measured after exclusion of particles having a particle size of less than 1µm.

In one aspect D50 is measured after exclusion of particles having a particle size of less than 1µm and the aerosol has a D50 of from 2 to 6µm. In one aspect D50 is measured after exclusion of particles having a particle size of less than 1µm and the aerosol has a D50 of from 2.5 to 6µm. In one aspect D50 is measured after exclusion of particles having a particle size of less than 1µm and the aerosol has a D50 of from 3 to 6µm. In one aspect D50 is measured after exclusion of particles having a particle size of less than 1µm and the aerosol has a D50 of from 3.5 to 6µm. In one aspect D50 is measured after exclusion of particles having a particle size of less than 1µm and the aerosol has a D50 of from 4 to 6µm. In one aspect D50 is measured after exclusion of particles having a particle size of less than 1µm and the aerosol has a D50 of from 4.5 to 6µm. In one aspect D50 is measured after exclusion of particles having a particle size of less than 1µm and the aerosol has a D50 of from 5 to 6µm. In one aspect D50 is measured after exclusion of particles having a particle size of less than 1µm and the aerosol has a D50 of from 2.5 to 5.5µm. In one aspect D50 is measured after exclusion of particles having a particle size of less than 1µm and the aerosol has a D50 of from 3 to 5.5µm. In one aspect D50 is measured after exclusion of particles having a particle size of less than 1µm and the aerosol has a D50 of from 3.5 to 5.5µm. In one aspect D50 is measured after exclusion of particles having a particle size of less than 1µm and the aerosol has a D50 of from 4 to 5.5µm. In one aspect D50 is measured after exclusion of particles having a particle size of less than 1µm and the aerosol has a D50 of from 4.5 to 5.5µm. In one aspect D50 is measured after exclusion of particles having a particle size of less than 1µm and the aerosol has a D50 of from 5 to 5.5µm. In one aspect D50 is measured after exclusion of particles having a particle size of less than 1µm and the aerosol has a D50 of from 4 to 5.5µm. In one aspect D50 is measured after exclusion of particles having a particle size of less than 1µm and the aerosol has a D50 of from 4 to 5µm. In one aspect D50 is measured after exclusion of particles having a particle size of less than 1µm and the aerosol has a D50 of from 4 to 4.5µm. In one aspect D50 is measured after exclusion of particles having a particle size of less than 1µm and the aerosol has a D50 of from 2.5 to 5µm. In one aspect D50 is measured after exclusion of particles having a particle size of less than 1µm and the aerosol has a D50 of from 2.5 to 4.5µm.

In one aspect D10 is measured after exclusion of particles having a particle size of less than 1µm and the aerosol has a D10 of at least 0.5µm. In one aspect D10 is measured after exclusion of particles having a particle size of less than 1µm and the aerosol has a D10 of at least 1µm. In one aspect D10 is measured after exclusion of particles having a particle size of less than 1µm and the aerosol has a D10 of at least 1.5µm. In one aspect D10 is measured after exclusion of particles having a particle size of less than 1µm and the aerosol has a D10 of at least 2µm.

In one aspect D90 is measured after exclusion of particles having a particle size of less than 1µm and the aerosol has a D90 of no greater than 15µm. In one aspect D90 is measured after exclusion of particles having a particle size of less than 1µm and the aerosol has a D90 of no greater than 12µm. In one aspect D90 is measured after exclusion of particles having a particle size of less than 1µm and the aerosol has a D90 of no greater than 10µm.

In one aspect D50 is measured after exclusion of particles having a particle size of greater than 30µm. In one aspect D10 is measured after exclusion of particles having a particle size of greater than 30µm. In one aspect D90 is measured after exclusion of particles having a particle size of greater than 30µm.

In one aspect D50 is measured after exclusion of particles having a particle size of greater than 30µm and the aerosol has a D50 of from 2 to 6µm. In one aspect D50 is measured after exclusion of particles having a particle size of greater than 30µm and the aerosol has a D50 of from 2.5 to 6µm. In one aspect D50 is measured after exclusion of particles having a particle size of greater than 30µm and the aerosol has a D50 of from 3 to 6µm. In one aspect D50 is measured after exclusion of particles having a particle size of greater than 30µm and the aerosol has a D50 of from 3.5 to 6µm. In one aspect D50 is measured after exclusion of particles having a particle size of greater than 30µm and the aerosol has a D50 of from 4 to 6µm. In one aspect D50 is measured after exclusion of particles having a particle size of greater than 30µm and the aerosol has a D50 of from 4.5 to 6µm. In one aspect D50 is measured after exclusion of particles having a particle size of greater than 30µm and the aerosol has a D50 of from 5 to 6µm. In one aspect D50 is measured after exclusion of particles having a particle size of greater than 30µm and the aerosol has a D50 of from 2.5 to 5.5µm. In one aspect D50 is measured after exclusion of particles having a particle size of greater than 30µm and the aerosol has a D50 of from 3 to 5.5µm. In one aspect D50 is measured after exclusion of particles having a particle size of greater than 30µm and the aerosol has a D50 of from 3.5 to 5.5µm. In one aspect D50 is measured after exclusion of particles having a particle size of greater than 30µm and the aerosol has a D50 of from 4 to 5.5µm. In one aspect D50 is measured after exclusion of particles having a particle size of greater than 30µm and the aerosol has a D50 of from 4.5 to 5.5µm. In one aspect D50 is measured after exclusion of particles having a particle size of greater than 30µm and the aerosol has a D50 of from 5 to 5.5µm. In one aspect D50 is measured after exclusion of particles having a particle size of greater than 30µm and the aerosol has a D50 of from 4 to 5.5µm. In one aspect D50 is measured after exclusion of particles having a particle size of greater than 30µm and the aerosol has a D50 of from 4 to 5µm. In one aspect D50 is measured after exclusion of particles having a particle size of greater than 30µm and the aerosol has a D50 of from 4 to 4.5µm. In one aspect D50 is measured after exclusion of particles having a particle size of greater than 30µm and the aerosol has a D50 of from 2.5 to 5µm. In one aspect D50 is measured after exclusion of particles having a particle size of greater than 30µm and the aerosol has a D50 of from 2.5 to 4.5µm.

In one aspect D10 is measured after exclusion of particles having a particle size of greater than 30µm and the aerosol has a D10 of at least 0.5µm. In one aspect D10 is measured after exclusion of particles having a particle size of greater than 30µm and the aerosol has a D10 of at least 1µm. In one aspect D10 is measured after exclusion of particles having a particle size of greater than 30µm and the aerosol has a D10 of at least 1.5µm. In one aspect D10 is measured after exclusion of particles having a particle size of greater than 30µm and the aerosol has a D10 of at least 2µm.

In one aspect D90 is measured after exclusion of particles having a particle size of greater than 30µm and the aerosol has a D90 of no greater than 15µm. In one aspect D90 is measured after exclusion of particles having a particle size of greater than 30µm and the aerosol has a D90 of no greater than 12µm. In one aspect D90 is measured after exclusion of particles having a particle size of greater than 30µm and the aerosol has a D90 of no greater than 10µm. In one aspect D90 is measured after exclusion of particles having a particle size of greater than 30µm and the aerosol has a D90 is from 10 to 30µm.

### Acid

As discussed above, the aerosolisable formulation and the aerosolised formulation of the present invention may contain one or more further components. These components may be selected depending on the nature of the formulation. In one aspect, the aerosolisable formulation and the aerosolised formulation further comprises an acid

In one aspect the acid is an organic acid. In one aspect the acid is a carboxylic acid. In one aspect the acid is an organic carboxylic acid.

In one aspect the acid is selected from the group consisting of acetic acid, lactic acid, formic acid, citric acid, benzoic acid, pyruvic acid, levulinic acid, succinic acid, tartaric acid, sorbic acid, propionic acid, phenylacetic acid, and mixtures thereof. In one aspect the acid is selected from the group consisting of citric acid, benzoic acid, levulinic acid, lactic acid, sorbic acid, and mixtures thereof. In one aspect the acid is selected from the group consisting of citric acid, benzoic acid, levulinic acid, and mixtures thereof. In one aspect the acid is at least citric acid. In one aspect the acid consists of citric acid.

In one aspect the acid is selected from acids having a pka of from 2 to 5. In one aspect the acid is a weak acid. In one aspect the acid is a weak organic acid.

In one aspect the acid has a solubility in water of at least 5g/L at 20 °C. In one aspect the acid has a solubility in water of at least 10g/L at 20 °C. In one aspect the acid has a solubility in water of at least 20g/L at 20 °C. In one aspect the acid has a solubility in water of at least 50g/L at 20 °C. In one aspect the acid has a solubility in water of at least 100g/L at 20 °C. In one aspect the acid has a solubility in water of at least 200g/L at 20 °C. In one aspect the acid has a solubility in water of at least 300g/L at 20 °C. In one aspect the acid has a solubility in water of at least 400g/L at 20 °C. In one aspect the acid has a solubility in water of at least 500g/L at 20 °C. In one aspect the acid has a solubility in water of at least 600g/L at 20 °C. In one aspect the acid has a solubility in water of at least 700g/L at 20 °C. In one aspect the acid has a solubility in water of at least 800g/L at 20 °C. In one aspect the acid has a solubility in water of at least 900g/L at 20 °C. In one aspect the acid has a solubility in water of at least 1000g/L at 20 °C. In one aspect the acid has a solubility in water of at least 1100g/L at 20 °C.

The molar ratio of acid to active agent (such as nicotine) may be selected as desired. In one aspect the molar ratio of acid to active agent (such as nicotine) is from 5:1 to 1:5. In one aspect the molar ratio of acid to active agent (such as nicotine) is from 4:1 to 1:4. In one aspect the molar ratio of acid to active agent (such as nicotine) is from 3:1 to 1:3. In one aspect the molar ratio of acid to active agent (such as nicotine) is from 2:1 to 1:2. In one aspect the molar ratio of acid to active agent (such as nicotine) is from 1.5:1 to 1:1.5. In one aspect the molar ratio of acid to active agent (such as nicotine) is from 1.2:1 to 1:1.2. In one aspect the molar ratio of acid to active agent (such as nicotine) is from 5:1 to 1:1. In one aspect the molar ratio of acid to active agent (such as nicotine) is from 4:1 to 1:1. In one aspect the molar ratio of acid to active agent (such as nicotine) is from 3:1 to 1:1. In one aspect the molar ratio of acid to active agent (such as nicotine) is from 2:1 to 1:1. In one aspect the molar ratio of acid to active agent (such as nicotine) is from 1.5:1 to 1:1. In one aspect the molar ratio of acid to active agent (such as nicotine) is from 1.2:1 to 1:1.

In one aspect the total content of acid present in the formulation is no greater than 5 mole equivalents based on the active agent (such as nicotine). In one aspect the total content of acid present in the formulation is no greater than 4 mole equivalents based on the active agent (such as nicotine). In one aspect the total content of acid present in the formulation is no greater than 3 mole equivalents based on the active agent (such as nicotine). In one aspect the total content of acid present in the formulation is no greater than 2 mole equivalents based on the active agent (such as nicotine). In one aspect the total content of acid present in the formulation is no greater than 1 mole equivalents based on the active agent (such as nicotine).

In one aspect the total content of acid present in the formulation is no less than 0.01 mole equivalents based on the active agent (such as nicotine). In one aspect the total content of acid present in the formulation is no less than 0.05 mole equivalents based on the active agent (such as nicotine). In one aspect the total content of acid present in the formulation is no less than 0.1 mole equivalents based on the active agent (such as nicotine). In one aspect the total content of acid present in the formulation is no less than 0.2 mole equivalents based on the active agent (such as nicotine). In one aspect the total content of acid present in the formulation is no less than 0.3 mole equivalents based on the active agent (such as nicotine). In one aspect the total content of acid present in the formulation is no less than 0.4 mole equivalents based on the active agent (such as nicotine). In one aspect the total content of acid present in the formulation is no less than 0.5 mole equivalents based on the active agent (such as nicotine). In one aspect the total content of acid present in the formulation is no less than 0.7 mole equivalents based on the active agent (such as nicotine).

In one aspect the acid is present in an amount of no greater than 6 wt% based on the aerosolisable formulation. In one aspect the acid is present in an amount of from 0.01 to 6 wt% based on the aerosolisable formulation. In one aspect the acid is present in an amount of from 0.02 to 6 wt% based on the aerosolisable formulation. In one aspect the acid is present in an amount of from 0.05 to 6 wt% based on the aerosolisable formulation. In one aspect the acid is present in an amount of from 0.08 to 6 wt% based on the aerosolisable formulation. In one aspect the acid is present in an amount of from 0.01 to 5 wt% based on the aerosolisable formulation. In one aspect the acid is present in an amount of from 0.02 to 5 wt% based on the aerosolisable formulation. In one aspect the acid is present in an amount of from 0.05 to 5 wt% based on the aerosolisable formulation. In one aspect the acid is present in an amount of from 0.08 to 5 wt% based on the aerosolisable formulation. In one aspect the acid is present in an amount of no greater than 4 wt% based on the aerosolisable formulation. In one aspect the acid is present in an amount of from 0.01 to 4 wt% based on the aerosolisable formulation. In one aspect the acid is present in an amount of from 0.02 to 4 wt% based on the aerosolisable formulation. In one aspect the acid is present in an amount of from 0.05 to 4 wt% based on the aerosolisable formulation. In one aspect the acid is present in an amount of from 0.08 to 4 wt% based on the aerosolisable formulation. In one aspect the acid is present in an amount of no greater than 3 wt% based on the aerosolisable formulation. In one aspect the acid is present in an amount of from 0.01 to 3 wt% based on the aerosolisable formulation. In one aspect the acid is present in an amount of from 0.02 to 3 wt% based on the aerosolisable formulation. In one aspect the acid is present in an amount of from 0.05 to 3 wt% based on the aerosolisable formulation. In one aspect the acid is present in an amount of from 0.08 to 3 wt% based on the aerosolisable formulation. In one aspect the acid is present in an amount of no greater than 2 wt% based on the aerosolisable formulation. In one aspect the acid is present in an amount of from 0.01 to 2 wt% based on the aerosolisable formulation. In one aspect the acid is present in an amount of from 0.02 to 2 wt% based on the aerosolisable formulation. In one aspect the acid is present in an amount of from 0.05 to 2 wt% based on the aerosolisable formulation. In one aspect the acid is present in an amount of from 0.08 to 2 wt% based on the aerosolisable formulation. In one aspect the acid is present in an amount of no greater than 1 wt% based on the aerosolisable formulation. In one aspect the acid is present in an amount of from 0.01 to 1 wt% based on the aerosolisable formulation. In one aspect the acid is present in an amount of from 0.02 to 1 wt% based on the aerosolisable formulation. In one aspect the acid is present in an amount of from 0.05 to 1 wt% based on the aerosolisable formulation. In one aspect the acid is present in an amount of from 0.08 to 1 wt% based on the aerosolisable formulation. In one aspect the acid is present in an amount of from 0.1 to 1 wt% based on the aerosolisable formulation. In one aspect the acid is present in an amount of no greater than 0.6 wt% based on the aerosolisable formulation. In one aspect the acid is present in an amount of from 0.01 to 0.6 wt% based on the aerosolisable formulation. In one aspect the acid is present in an amount of from 0.02 to 0.6 wt% based on the aerosolisable formulation. In one aspect the acid is present in an amount of from 0.05 to 0.6 wt% based on the aerosolisable formulation. In one aspect the acid is present in an amount of from 0.08 to 0.6 wt% based on the aerosolisable formulation. In one aspect the acid is present in an amount of from 0.1 to 0.6 wt% based on the aerosolisable formulation. In one aspect the acid is present in an amount of no greater than 0.5 wt% based on the aerosolisable formulation. In one aspect the acid is present in an amount of from 0.01 to 0.5 wt% based on the aerosolisable formulation. In one aspect the acid is present in an amount of from 0.02 to 0.5 wt% based on the aerosolisable formulation. In one aspect the acid is present in an amount of from 0.05 to 0.5 wt% based on the aerosolisable formulation. In one aspect the acid is present in an amount of from 0.08 to 0.5 wt% based on the aerosolisable formulation. In one aspect the acid is present in an amount of no greater than 0.2 wt% based on the aerosolisable formulation. In one aspect the acid is present in an amount of from 0.01 to 0.2 wt% based on the aerosolisable formulation. In one aspect the acid is present in an amount of from 0.02 to 0.2 wt% based on the aerosolisable formulation. In one aspect the acid is present in an amount of from 0.05 to 0.2 wt% based on the aerosolisable formulation. In one aspect the acid is present in an amount of from 0.08 to 0.2 wt% based on the aerosolisable formulation. In one aspect the acid is present in an amount of no greater than 0.1 wt% based on the aerosolisable formulation. In one aspect the acid is present in an amount of from 0.01 to 0.1 wt% based on the aerosolisable formulation. In one aspect the acid is present in an amount of from 0.02 to 0.1 wt% based on the aerosolisable formulation. In one aspect the acid is present in an amount of from 0.05 to 0.1 wt% based on the aerosolisable formulation. In one aspect the acid is present in an amount of from 0.08 to 0.1 wt% based on the aerosolisable formulation.

The amount of acid and the solubility of the acid may be selected such that a given amount of the acid will dissolve in the water. In one aspect at 20 °C at least 20% of the acid dissolves in the water. In one aspect at 25 °C at least 20% of the acid dissolves in the water. In one aspect at 30 °C at least 20% of the acid dissolves in the water. In one aspect at 20 °C at least 35% of the acid dissolves in the water. In one aspect at 20 °C at least 40% of the acid dissolves in the water. In one aspect at 20 °C at least 45% of the acid dissolves in the water. In one aspect at 20 °C at least 50% of the acid dissolves in the water. In one aspect at 20 °C at least 55% of the acid dissolves in the water.

As is understood by one skilled in the art, nicotine may exist in unprotonated form, monoprotonated form or diprotonated form. The structures of each of these forms are given below.

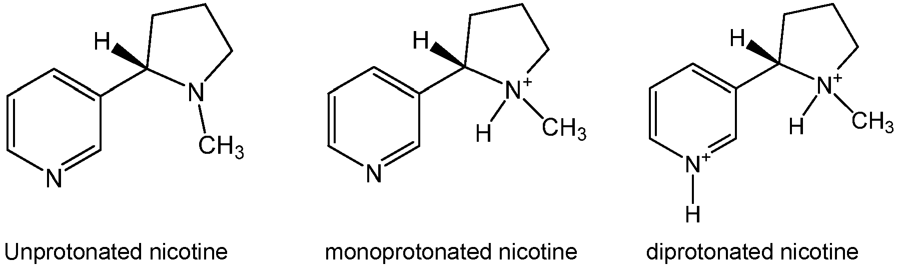

Reference in the specification to protonated form means both monoprotonated nicotine and diprotonated nicotine. Reference in the specification to amounts in the protonated form means the combined amount of monoprotonated nicotine and diprotonated nicotine. Furthermore, when reference is made to a fully protonated formulation it will be understood that at any one time there may be very minor amounts of unprotonated nicotine present, e.g. less than 1% unprotonated.

The formulation may comprise nicotine in protonated form. The formulation may comprise nicotine in unprotonated form. In one aspect the formulation comprises nicotine in unprotonated form and nicotine in monoprotonated form. In one aspect the formulation comprises nicotine in unprotonated form and nicotine in diprotonated form. In one aspect the formulation comprises nicotine in unprotonated form, nicotine in monoprotonated form and nicotine in diprotonated form.

In one aspect at least 5wt% of the nicotine present in the formulation is in protonated form. In one aspect at least 10wt% of the nicotine present in the formulation is in protonated form. In one aspect at least 15wt% of the nicotine present in the formulation is in protonated form. In one aspect at least 20wt% of the nicotine present in the formulation is in protonated form. In one aspect at least 25wt% of the nicotine present in the formulation is in protonated form. In one aspect at least 30wt% of the nicotine present in the formulation is in protonated form. In one aspect at least 35wt% of the nicotine present in the formulation is in protonated form. In one aspect at least 40wt% of the nicotine present in the formulation is in protonated form. In one aspect at least 45wt% of the nicotine present in the formulation is in protonated form. In one aspect at least 50wt% of the nicotine present in the formulation is in protonated form. In one aspect at least 55wt% of the nicotine present in the formulation is in protonated form. In one aspect at least 60wt% of the nicotine present in the formulation is in protonated form. In one aspect at least 65wt% of the nicotine present in the formulation is in protonated form. In one aspect at least 70wt% of the nicotine present in the formulation is in protonated form. In one aspect at least 75wt% of the nicotine present in the formulation is in protonated form. In one aspect at least 80wt% of the nicotine present in the formulation is in protonated form. In one aspect at least 85wt% of the nicotine present in the formulation is in protonated form. In one aspect at least 90wt% of the nicotine present in the formulation is in protonated form. In one aspect at least 95wt% of the nicotine present in the formulation is in protonated form. In one aspect at least 99wt% of the nicotine present in the formulation is in protonated form. In one aspect at least 99.9wt% of the nicotine present in the formulation is in protonated form.

In one aspect from 50 to 95 wt% of the nicotine present in the formulation is in protonated form. In one aspect from 55 to 95 wt% of the nicotine present in the formulation is in protonated form. In one aspect from 60 to 95 wt% of the nicotine present in the formulation is in protonated form. In one aspect from 65 to 95 wt% of the nicotine present in the formulation is in protonated form. In one aspect from 70 to 95 wt% of the nicotine present in the formulation is in protonated form. In one aspect from 75 to 95 wt% of the nicotine present in the formulation is in protonated form. In one aspect from 80 to 95 wt% of the nicotine present in the formulation is in protonated form. In one aspect from 85 to 95 wt% of the nicotine present in the formulation is in protonated form. In one aspect from 90 to 95 wt% of the nicotine present in the formulation is in protonated form.

The relevant amounts of nicotine which are present in the formulation in protonated form are specified herein. These amounts may be readily calculated by one skilled in the art. Nicotine, 3-(1-methylpyrrolidin-2-yl) pyridine, is a diprotic base with pKa of 3.12 for the pyridine ring and 8.02 for the pyrrolidine ring It can exist in pH-dependent protonated (mono- and di-) and non-protonated (free base) forms which have different bioavailability.

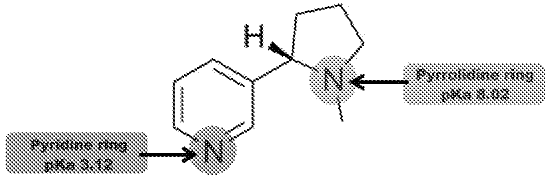

The distribution of protonated and non-protonated nicotine will vary at various pH increments.

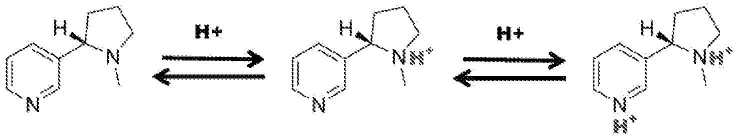

The fraction of non-protonated nicotine will be predominant at high pH levels whilst a decrease in the pH will see an increase of the fraction of protonated nicotine (mono- or di-depending on the pH). If the relative fraction of protonated nicotine and the total amount of nicotine in the sample are known, the absolute amount of protonated nicotine can be calculated.

The relative fraction of protonated nicotine in formulation can be calculated by using the Henderson-Hasselbalch equation, which describes the pH as a derivation of the acid dissociation constant equation, and it is extensively employed in chemical and biological systems. Consider the following equilibrium:

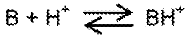

The Henderson-Hasselbalch equation for this equilibrium is: $pH = pKa + log \frac{\left[B\right]}{\left[BH+\right]}$

Where [B] is the amount of non-protonated nicotine (i.e. free base), [BH+] the amount of protonated nicotine (i.e. conjugate acid) and pKa is the reference pKa value for the pyrrolidine ring nitrogen of nicotine (pKa=8.02). The relative fraction of protonated nicotine can be derived from the alpha value of the non-protonated nicotine calculated from the Henderson-Hasselbalch equation as: $% protonated nicotine = 100 - \left\{\frac{\frac{\left[B\right]}{\left[BH+\right]}}{\left\{1+\frac{\left[B\right]}{\left[BH+\right]}\right\}}∗100\right\}$

Determination of pKa values of nicotine formulations was carried out using the basic approach described in "Spectroscopic investigations into the acid-base properties of nicotine at different temperatures", Peter M. Clayton, Carl A. Vas, Tam T. T. Bui, Alex F. Drake and Kevin McAdam, .Anal. Methods, 2013,5, 81-88.

### Flavour

The aerosolisable formulation and the aerosolised formulation of the present invention may contain one or more further components. These components may be selected depending on the nature of the formulation. The aerosolisable formulation may comprise one or more flavours or flavouring components. As used herein, the terms "flavour" and "flavourant" refer to materials which, where local regulations permit, may be used to create a desired taste or aroma in a product for adult consumers. They may include extracts (e.g. liquorice, hydrangea, Japanese white bark magnolia leaf, chamomile, fenugreek, clove, menthol, Japanese mint, aniseed, cinnamon, herb, wintergreen, cherry, berry, peach, apple, Drambuie, bourbon, scotch, whiskey, spearmint, peppermint, lavender, cardamom, celery, cascarilla, nutmeg, sandalwood, bergamot, geranium, honey essence, rose oil, vanilla, lemon oil, orange oil, cassia, caraway, cognac, jasmine, ylang-ylang, sage, fennel, piment, ginger, anise, coriander, coffee, or a mint oil from any species of the genus Mentha), flavour enhancers, bitterness receptor site blockers, sensorial receptor site activators or stimulators, sugars and/or sugar substitutes (e.g., sucralose, acesulfame potassium, aspartame, saccharine, cyclamates, lactose, sucrose, glucose, fructose, sorbitol, or mannitol), and other additives such as charcoal, chlorophyll, minerals, botanicals, or breath freshening agents. They may be imitation, synthetic or natural ingredients or blends thereof. They may be in any suitable form, for example, oil, liquid, or powder. The one or more flavours may be selected from the group consisting of (4-(para-)methoxyphenyl)-2-butanone, vanillin, γ-undecalactone, menthone, 5-propenyl guaethol, menthol, para-mentha-8-thiol-3-one and mixtures thereof. In one aspect the flavour is at least menthol.

If present, the one or more flavours may be present in any suitable amount. In one aspect the one or more flavours are present in a total amount of no greater than 10 wt.% based on the aerosolisable formulation. In one aspect the one or more flavours are present in a total amount of no greater than 7 wt.% based on the aerosolisable formulation. In one aspect the one or more flavours are present in a total amount of no greater than 5 wt.% based on the aerosolisable formulation. In one aspect the one or more flavours are present in a total amount of no greater than 4 wt.% based on the aerosolisable formulation. In one aspect the one or more flavours are present in a total amount of no greater than 3 wt.% based on the aerosolisable formulation. In one aspect the one or more flavours are present in a total amount of no greater than 2wt.% based on the aerosolisable formulation. In one aspect the one or more flavours are present in a total amount of no greater than 1wt.% based on the aerosolisable formulation.

In one aspect the one or more flavours are present in a total amount of from 0.01 to 5wt.% based on the aerosolisable formulation. In one aspect the one or more flavours are present in a total amount of from 0.01 to 4wt.% based on the aerosolisable formulation. In one aspect the one or more flavours are present in a total amount of from 0.01 to 3wt.% based on the aerosolisable formulation. In one aspect the one or more flavours are present in a total amount of from 0.01 to 2wt.% based on the aerosolisable formulation. In one aspect the one or more flavours are present in a total amount of from 0.01 to 1wt.% based on the aerosolisable formulation. In one aspect the one or more flavours are present in a total amount of from 0.01 to 0.5wt.% based on the aerosolisable formulation.

In one aspect the one or more flavours are present in a total amount of no greater than 10 wt.% based on the aerosolised formulation. In one aspect the one or more flavours are present in a total amount of no greater than 7 wt.% based on the aerosolised formulation. In one aspect the one or more flavours are present in a total amount of no greater than 5 wt.% based on the aerosolised formulation. In one aspect the one or more flavours are present in a total amount of no greater than 4 wt.% based on the aerosolised formulation. In one aspect the one or more flavours are present in a total amount of no greater than 3 wt.% based on the aerosolised formulation. In one aspect the one or more flavours are present in a total amount of no greater than 2wt.% based on the aerosolised formulation. In one aspect the one or more flavours are present in a total amount of no greater than 1wt.% based on the aerosolised formulation.

In one aspect the one or more flavours are present in a total amount of from 0.01 to 5wt.% based on the aerosolised formulation. In one aspect the one or more flavours are present in a total amount of from 0.01 to 4wt.% based on the aerosolised formulation. In one aspect the one or more flavours are present in a total amount of from 0.01 to 3wt.% based on the aerosolised formulation. In one aspect the one or more flavours are present in a total amount of from 0.01 to 2wt.% based on the aerosolised formulation. In one aspect the one or more flavours are present in a total amount of from 0.01 to 1wt.% based on the aerosolised formulation. In one aspect the one or more flavours are present in a total amount of from 0.01 to 0.5wt.% based on the aerosolised formulation.

### Formulation

One or more cyclodextrins may or may not be present in any suitable amount in the aerosolisable formulation. In one aspect the one or more cyclodextrins are present in a total amount of no greater than 12 wt.% based on the aerosolisable formulation. In one aspect the one or more cyclodextrins are present in a total amount of no greater than 10 wt.% based on the aerosolisable formulation. In one aspect the one or more cyclodextrins are present in a total amount of no greater than 9 wt.% based on the aerosolisable formulation. In one aspect the one or more cyclodextrins are present in a total amount of no greater than 8 wt.% based on the aerosolisable formulation. In one aspect the one or more cyclodextrins are present in a total amount of no greater than 7 wt.% based on the aerosolisable formulation. In one aspect the one or more cyclodextrins are present in a total amount of no greater than 6 wt.% based on the aerosolisable formulation. In one aspect the one or more cyclodextrins are present in a total amount of no greater than 5 wt.% based on the aerosolisable formulation. In one aspect the one or more cyclodextrins are present in a total amount of no greater than 4 wt.% based on the aerosolisable formulation. In one aspect the one or more cyclodextrins are present in a total amount of no greater than 3 wt.% based on the aerosolisable formulation. In one aspect the one or more cyclodextrins are present in a total amount of no greater than 2 wt.% based on the aerosolisable formulation. In one aspect the one or more cyclodextrins are present in a total amount of no greater than 1 wt.% based on the aerosolisable formulation. In one aspect the one or more cyclodextrins are present in a total amount of no greater than 0.5 wt.% based on the aerosolisable formulation. In one aspect the one or more cyclodextrins are present in a total amount of no greater than 0.1 wt.% based on the aerosolisable formulation. In one aspect the one or more cyclodextrins are present in a total amount of no greater than 0.01 wt.% based on the aerosolisable formulation. In one aspect the one or more cyclodextrins are present in a total amount of no greater than 0.001 wt.% based on the aerosolisable formulation.

One or more cyclodextrins may or may not be present in any suitable amount in the aerosolised formulation. In one aspect the one or more cyclodextrins are present in a total amount of no greater than 12 wt.% based on the aerosolised formulation. In one aspect the one or more cyclodextrins are present in a total amount of no greater than 10 wt.% based on the aerosolised formulation. In one aspect the one or more cyclodextrins are present in a total amount of no greater than 9 wt.% based on the aerosolised formulation. In one aspect the one or more cyclodextrins are present in a total amount of no greater than 8 wt.% based on the aerosolised formulation. In one aspect the one or more cyclodextrins are present in a total amount of no greater than 7 wt.% based on the aerosolised formulation. In one aspect the one or more cyclodextrins are present in a total amount of no greater than 6 wt.% based on the aerosolised formulation. In one aspect the one or more cyclodextrins are present in a total amount of no greater than 5 wt.% based on the aerosolised formulation. In one aspect the one or more cyclodextrins are present in a total amount of no greater than 4 wt.% based on the aerosolised formulation. In one aspect the one or more cyclodextrins are present in a total amount of no greater than 3 wt.% based on the aerosolised formulation. In one aspect the one or more cyclodextrins are present in a total amount of no greater than 2 wt.% based on the aerosolised formulation. In one aspect the one or more cyclodextrins are present in a total amount of no greater than 1 wt.% based on the aerosolised formulation. In one aspect the one or more cyclodextrins are present in a total amount of no greater than 0.5 wt.% based on the aerosolised formulation. In one aspect the one or more cyclodextrins are present in a total amount of no greater than 0.1 wt.% based on the aerosolised formulation. In one aspect the one or more cyclodextrins are present in a total amount of no greater than 0.01 wt.% based on the aerosolised formulation. In one aspect the one or more cyclodextrins are present in a total amount of no greater than 0.001 wt.% based on the aerosolised formulation.

The one or more cyclodextrins may be selected from the group consisting of unsubstituted cyclodextrins, substituted cyclodextrins and mixtures thereof. In one aspect at least one cyclodextrin is an unsubstituted cyclodextrin. In one aspect the one or more cyclodextrins are selected from the group consisting of unsubstituted cyclodextrins. In one aspect at least one cyclodextrin is a substituted cyclodextrin. In one aspect the one or more cyclodextrins are selected from the group consisting of substituted cyclodextrins.

In one aspect the one or more cyclodextrins are selected from the group consisting of unsubstituted (α)-cyclodextrin, substituted (α)-cyclodextrin, unsubstituted (β)-cyclodextrin, substituted (β)-cyclodextrin, unsubstituted (γ)-cyclodextrin, substituted (γ)-cyclodextrin, and mixtures thereof. In one aspect the one or more cyclodextrins are selected from the group consisting of unsubstituted (β)-cyclodextrin, substituted (β)-cyclodextrin, and mixtures thereof.

In one aspect the one or more cyclodextrins are selected from the group consisting of unsubstituted (α)-cyclodextrin, unsubstituted (β)-cyclodextrin, unsubstituted (γ)-cyclodextrin, and mixtures thereof. In one aspect the one or more cyclodextrins is selected from unsubstituted (β)-cyclodextrin.

In one aspect the one or more cyclodextrins are selected from the group consisting of substituted (α)-cyclodextrin, substituted (β)-cyclodextrin, substituted (γ)-cyclodextrin, and mixtures thereof. In one aspect the one or more cyclodextrins is selected from substituted (β)-cyclodextrins. Chemical substitutions at the 2-, 3-, and 6-hydroxyl sites are envisaged, and in particular substitution at the 2-position.

In one aspect the one or more cyclodextrins are selected from the group consisting of 2-hydroxy-propyl-α-cyclodextrin, 2-hydroxy-propyl-β-cyclodextrin, 2-hydroxy-propyl-γ-cyclodextrin and mixtures thereof. In one aspect the one or more cyclodextrins is at least 2-hydroxy-propyl-α-cyclodextrin. In one aspect the one or more cyclodextrins is at least 2-hydroxy-propyl-β-cyclodextrin. In one aspect the one or more cyclodextrins is at least 2-hydroxy-propyl-γ-cyclodextrin.

2-hydroxy-propyl derivatives of cyclodextrins, such as 2-hydroxy-propyl-β-cyclodextrin have increased solubility in water when compared to base cyclodextrins such as β-cyclodextrin. In one aspect if the aerosolisable formulation contains one or more cyclodextrins, then the aerosolisable formulation contains no flavours that can be encapsulated by the one or more cyclodextrins.

### Process

As discussed herein, in one aspect there is provided a process for forming an aerosol, the process comprising aerosolising an aerosolisable formulation comprising
(i) water in an amount of at least 50 wt.% based on the aerosolisable formulation; and
(ii) at least one active agent;
to provide an aerosol having the following particle size distribution: D50 of from 3 to 6µm.

In the process the aerosol may be formed by a process performed at a temperature below 60°C.In the process the aerosol may be formed by a process performed at a temperature below 50°C. In the process the aerosol may be formed by a process performed at a temperature below 40°C. In the process the aerosol may be formed by a process performed at a temperature below 30°C. In the process the aerosol may be formed by a process performed at a temperature below 25°C. In the process the aerosol may be formed by a process which does not involve heating.

In the process the aerosol may be formed by applying ultrasonic energy to the aerosolisable formulation.

The formulation may be contained or delivered by any means. In one aspect the present invention provides a contained aerosolisable formulation comprising (a) one or more containers; and (b) an aerosolisable formulation as defined herein. The container may be any suitable container, for example to allow for the storage or delivery of the formulation. In one aspect the container is configured for engagement with an electronic aerosol provision system. The container may be configured to become fluidly in communication with an electronic aerosol provision system so that formulation may be delivered to the electronic aerosol provision system. As described above, the present disclosure relates to container which may be used in an electronic aerosol provision system, such as an e-cigarette. Throughout the following description the term "e-cigarette" is used; however, this term may be used interchangeably with electronic aerosol provision system.

As discussed herein, the container of the present invention is typically provided for the delivery of aerosolisable formulation to or within an e-cigarette. The aerosolisable formulation may be held within an e-cigarette or may be sold as a separate container for subsequent use with or in an e-cigarette. As understood by one skilled in the art, e-cigarettes may contain a unit known as a detachable cartomiser which typically comprises a reservoir of aerosolisable formulation, and an aerosoliser such as a wick material and a heating element for vaporising the aerosolisable formulation. In some e-cigarettes, the cartomiser is part of a single-piece device and is not detachable. In one aspect the container is a cartomiser or is part of a cartomiser. In one aspect the container is not a cartomiser or part of a cartomiser and is a container, such as a tank, which may be used to deliver nicotine formulation to or within an e-cigarette.

In one aspect the container is part of an e-cigarette. Therefore in a further aspect the present invention provides an electronic aerosol provision system comprising: an aerosolisable formulation as defined herein; an aerosoliser for aerosolising formulation for inhalation by a user of the electronic aerosol provision system; and a power supply comprising a cell or battery for supplying power to the aerosoliser.

In addition to the aerosolisable formulation of the present invention and to systems such as containers and electronic aerosol provision systems containing the same, the present invention provides a process for improving the sensory properties of an aerosolised nicotine. In a further aspect the present invention provides a process for improving the storage stability of an aerosolised nicotine formulation.

Reference to an improvement in the sensory properties of a vaporised nicotine solution refer may include an improvement in the smoothness of the vaporised nicotine solution as perceived by a user.

The process of the present invention may comprises additional steps either before the steps listed, after the steps listed or between one or more of the steps listed.

### BRIEF DESCRIPTION OF THE DRAWINGS

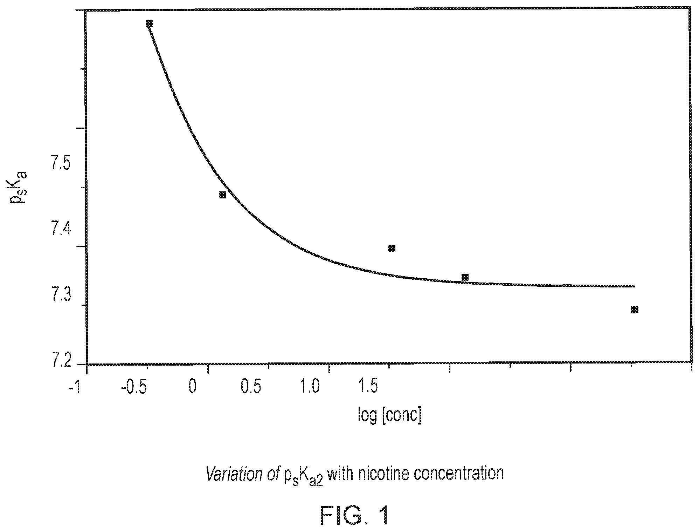

The present invention will now be described in further detail by way of example only with reference to the accompanying figure in which:-
Figure 1 shows a graph illustrating variation of pₛKₐ₂ with nicotine concentration; and

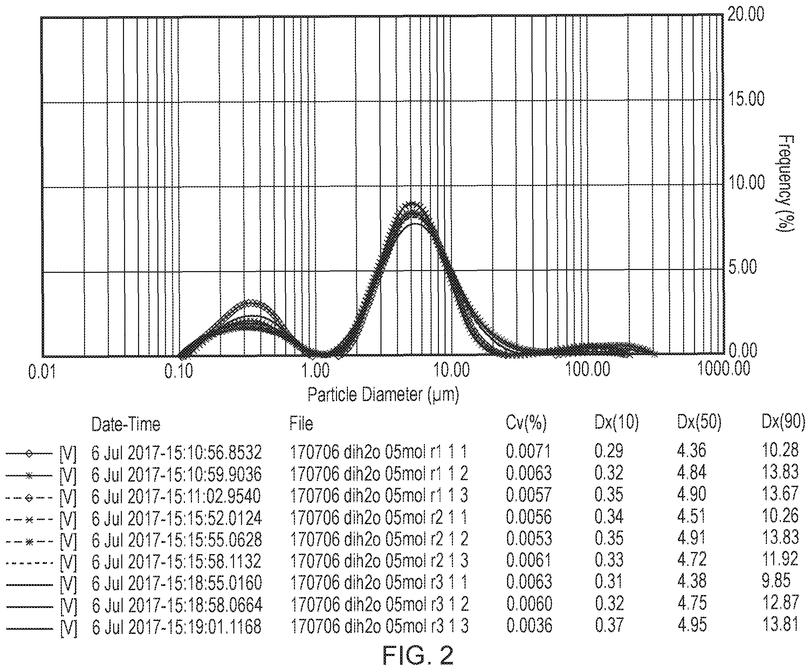

Figure 2 shows particle size distribution of the present aerosolised formulation.

The invention will now be described with reference to the following non-limiting example.

### Examples

A series of tests were conducted using a commercially available vibrating mesh nebuliser device. The device was loaded with formulation containing 90.0% (w/w) water, 9.0% 2-hydroxy-propyl-β-cyclodextrin, 0.9% I-menthol, and 0.1% nicotine.

The Droplet Size Distribution (DSD) formed on actuation of the device was determined primarily by laser diffraction. For example, the Malvern Instruments (Worcestershire, UK) Spraytec system uses these principles to determine the droplet size distribution of aerosols in the range from 0.1 - 900 µm.

The position of the aerosol source (e.g. the Device mouthpiece) was kept constant and at a fixed distance from the laser beam (2.5 cm) for the duration of the measurements, and to ensure appropriate actuation into the path of the laser beam. Data collection was triggered by the change in light transmittance as droplets entered the measurement zone and continued over 3 seconds at a sampling rate of 2.5 kHz.

Both the British Pharmacopoeia Commission (2014) [British Pharmacopoeia. London, England: Stationery Office] and The Council of Europe (2013) [European Pharmacopoeia. Strasbourg, France: Council of Europe] have published standards relating to the validity of Laser Diffraction procedures as a relevant test methodology for medicinal substances, including nicotine. Acceptance criteria for x₅₀ data (central distribution value) are specified as Sᵣₑₗ ≤ 10% over at least 6 replicates. Additionally, the acceptance criteria for *x*₁₀ and *x*₉₀ data (values at the sides of the distribution) are specified as Sᵣₑₗ ≤ 15% over at least 6 replicates. It should be noted that these conventions include the caveat that the criteria must be doubled for values below 10 µm in size.

It is recognised that Laser Diffraction techniques are more widely available that alternative techniques (e.g. cascade impactors) and have a longer history of use for product testing for inhaled aerosols.

Various modifications and variations of the present invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in chemistry or related fields are intended to be within the scope of the following claims.

The present disclosure will now be further defined in the following paragraphs:
1. An aerosolised formulation comprising
   (i) water in an amount of at least 50 wt.% based on the aerosolised formulation; and
   (ii) at least one active agent;
   wherein the aerosol has the following particle size distribution: D50 of from 2 to 6µm.
2. An aerosolised formulation according to paragraph 1 wherein water is present in an amount of at least 75 wt.% based on the aerosolised formulation.
3. An aerosolised formulation according to paragraph 1 or 2 wherein water is present in an amount of at least 90 wt.% based on the aerosolised formulation.
4. An aerosolised formulation according to any one of paragraphs 1 to 3 wherein the active agent is present in an amount of no greater than 1 wt.% based on the aerosolised formulation.
5. An aerosolised formulation according to paragraph 4 wherein the active agent is present in an amount of from 0.01 to 0.6 wt.% based on the aerosolised formulation.
6. An aerosolised formulation according to any one of paragraphs 1 to 5 wherein the active agent is at least nicotine.
7. An aerosolised formulation according to any one of paragraphs 1 to 6 wherein the aerosol has a D50 of from 3.5 to 5.5µm.
8. An aerosolised formulation according to any one of paragraphs 1 to 7 wherein the aerosol has a D50 of from 4.5 to 5.5µm.
9. An aerosolised formulation according to any one of paragraphs 1 to 8 wherein the aerosol has a D10 of at least 0.5µm.
10. An aerosolised formulation according to any one of paragraphs 1 to 9 wherein the aerosol has a D90 of no greater than 15µm.
11. An aerosolised formulation according to any one of paragraphs 1 to 10 wherein the aerosolised formulation further comprises an acid
12. An aerosolised formulation according to paragraph 11 wherein the acid is selected from the group consisting of acetic acid, lactic acid, formic acid, citric acid, benzoic acid, pyruvic acid, levulinic acid, succinic acid, tartaric acid, sorbic acid, propionic acid, phenylacetic acid, and mixtures thereof.
13. An aerosolised formulation according to paragraph 11 or 12 wherein the acid is selected from the group consisting of citric acid, benzoic acid, levulinic acid, sorbic acid, lactic acid, and mixtures thereof.
14. An aerosolised formulation according to any one of paragraphs 11to 13 wherein the acid is at least citric acid.
15. An aerosolised formulation according to any one of paragraphs 11 to 14 wherein the total content of acid present in the formulation is no greater than 1 mole equivalents based on the active agent.
16. An aerosolised formulation according to any one of paragraphs 11 to 15 wherein the total content of acid present in the solution is no less than 0.01 mole equivalents based on the active agent.
17. An aerosolised formulation according to any one of paragraphs 1 to 16 further comprising one or more flavours.
18. An aerosolised formulation according to paragraph 17 wherein the one or more flavours are selected from the group consisting of (4-(para-)methoxyphenyl)-2-butanone, vanillin, γ-undecalactone, menthone, 5-propenyl guaethol, menthol, para-mentha-8-thiol-3-one and mixtures thereof.
19. An aerosolised formulation according to paragraph 18 wherein the flavour is at least menthol.
20. An aerosolised formulation according to any one of paragraphs 17 to 19 wherein the one or more flavours are present in a total amount of no greater than 2wt.% based on the aerosolised formulation.
21. An aerosolised formulation according to any one of paragraphs 17 to 20 wherein the one or more flavours are present in a total amount of from 0.01 to 1wt.% based on the aerosolised formulation.
22. A process for forming an aerosol, the process comprising aerosolising an aerosolisable formulation comprising
   (i) water in an amount of at least 50 wt.% based on the aerosolisable formulation; and
   (ii) at least one active agent;
   to provide an aerosol having the following particle size distribution: D50 of from 3 to 6µm.
23. A process according to paragraph 22 wherein the aerosolisable formulation is a formulation as defined in any one of paragraphs 2 to 21.
24. A process according to paragraph 22 or 23 wherein the aerosol is formed by a process performed at a temperature below 50°C.
25. A process according to paragraph 22, 23 or 24 wherein the aerosol is formed by applying ultrasonic energy to the aerosolised formulation.
26. An electronic aerosol provision system comprising:
   (a) an aerosoliser for aerosolising formulation for inhalation by a user of the electronic aerosol provision system;
   (b) a power supply comprising a cell or battery for supplying power to the aerosoliser
   (c) aerosolisable formulation comprising
      (i) water in an amount of at least 50 wt.% based on the aerosolisable formulation; and
      (ii) at least one active agent;
   wherein the aerosoliser provides an aerosol having the following particle size distribution: D50 of from 2 to 6µm.
27. An electronic aerosol provision system according to paragraph 26 wherein the aerosolised formulation is an aerosolised formulation as defined in any one of paragraphs 2 to 21.
28. A process for improving delivery of an aerosolised formulation to the lungs, the process comprising the steps of
   (a) providing an aerosolisable material comprising (i) water in an amount of at least 50 wt.% based on the aerosolisable material and (ii) at least one active agent;
   (b) aerosolising the material aerosolisable material to provide an aerosol having the following particle size distribution: D50 of from 2 to 6µm.

## Claims

1. An aerosolised formulation comprising
(i) water in an amount of at least 50 wt.% based on the aerosolised formulation;
(ii) at least one active agent; and
(iii) an acid;
wherein the aerosol has the following particle size distribution: D50 of from 2 to 6µm.

2. An aerosolised formulation according to claim 1 wherein water is present in an amount of at least 75 wt.% based on the aerosolised formulation, such as wherein water is present in an amount of at least 90 wt.% based on the aerosolised formulation.

3. An aerosolised formulation according to any one of claims 1 or 2 wherein the active agent is present in an amount of no greater than 1 wt.% based on the aerosolised formulation, such as wherein the active agent is present in an amount of from 0.01 to 0.6 wt.% based on the aerosolised formulation.

4. An aerosolised formulation according to any one of claims 1 to 3 wherein the active agent is at least nicotine.

5. An aerosolised formulation according to any one of claims 1 to 4 wherein the aerosol has a D50 of from 3.5 to 5.5µm, such as wherein the aerosol has a D50 of from 4.5 to 5.5µm.

6. An aerosolised formulation according to any one of claims 1 to 5 wherein the aerosol has a D10 of at least 0.5µm and/or wherein the aerosol has a D90 of no greater than 15µm.

7. An aerosolised formulation according to any one or claims 1 to 6 wherein the acid is selected from the group consisting of acetic acid, lactic acid, formic acid, citric acid, benzoic acid, pyruvic acid, levulinic acid, succinic acid, tartaric acid, sorbic acid, propionic acid, phenylacetic acid, and mixtures thereof, such as wherein the acid is selected from the group consisting of citric acid, benzoic acid, levulinic acid, sorbic acid, lactic acid, and mixtures thereof, such as wherein the acid is at least citric acid.

8. An aerosolised formulation according to any one of claims 1 to 7 wherein the total content of acid present in the formulation is no greater than 1 mole equivalents based on the active agent, such as wherein the total content of acid present in the solution is no less than 0.01 mole equivalents based on the active agent.

9. An aerosolised formulation according to any one of claims 1 to 8 further comprising one or more flavours, such as wherein the one or more flavours are selected from the group consisting of (4-(para-)methoxyphenyl)-2-butanone, vanillin, γ-undecalactone, menthone, 5-propenyl guaethol, menthol, para-mentha-8-thiol-3-one and mixtures thereof, such as wherein the flavour is at least menthol.

10. An aerosolised formulation according to claim 9 wherein the one or more flavours are present in a total amount of no greater than 2wt.% based on the aerosolised formulation, such as wherein the one or more flavours are present in a total amount of from 0.01 to 1wt.% based on the aerosolised formulation.

11. A process for forming an aerosol, the process comprising aerosolising an aerosolisable formulation comprising
(i) water in an amount of at least 50 wt.% based on the aerosolisable formulation;
(ii) at least one active agent;
(iii) an acid;
to provide an aerosol having the following particle size distribution: D50 of from 3 to 6µm.

12. A process according to claim 11 wherein the aerosol is formed by a process performed at a temperature below 50°C.

13. A process according to claim 11 or 12 wherein the aerosol is formed by applying ultrasonic energy to the aerosolised formulation.

14. An electronic aerosol provision system comprising:
(a) an aerosoliser for aerosolising formulation for inhalation by a user of the electronic aerosol provision system;
(b) a power supply comprising a cell or battery for supplying power to the aerosoliser
(c) aerosolisable formulation comprising
(i) water in an amount of at least 50 wt.% based on the aerosolisable formulation;
(ii) at least one active agent; and
(iii) an acid;
wherein the aerosoliser provides an aerosol having the following particle size distribution: D50 of from 2 to 6µm.

15. A process for improving delivery of an aerosolised formulation to the lungs, the process comprising the steps of
(a) providing an aerosolisable material comprising (i) water in an amount of at least 50 wt.% based on the aerosolisable material, (ii) at least one active agent and (iii) an acid;
(b) aerosolising the material aerosolisable material to provide an aerosol having the following particle size distribution: D50 of from 2 to 6µm.
